(19) Europäisches Patentamt
European Patent Office
Office européen des brevets

(11) Veröffentlichungsnummer: **0 350 693 B1**

(12) **EUROPÄISCHE PATENTSCHRIFT**

(45) Veröffentlichungstag der Patentschrift: **28.09.94**

(51) Int. Cl.[5]: **C07D 205/04**, A01N 43/44

(21) Anmeldenummer: **89111542.0**

(22) Anmeldetag: **24.06.89**

# (54) Azetidinderivate, Verfahren zu ihrer Herstellung und ihre Verwendung zur Regulierung des Pflanzenwachstums.

(30) Priorität: **15.07.88 DE 3824052**
**19.05.89 DE 3916364**

(43) Veröffentlichungstag der Anmeldung:
**17.01.90 Patentblatt 90/03**

(45) Bekanntmachung des Hinweises auf die Patenterteilung:
**28.09.94 Patentblatt 94/39**

(84) Benannte Vertragsstaaten:
**AT BE CH DE ES FR GB IT LI NL SE**

(56) Entgegenhaltungen:
**DE-A- 2 312 045**
**DE-A- 2 457 688**
**US-A- 3 536 721**

**THE JOURNAL OF ORGANIC CHEMISTRY, Band 39, Nr. 15, 26. Juli 1974, Seiten 2264-2267; J.C. SHEEHAN et al.: "Oxidation of cyclic amines with rutheniumtetroxide"**

(73) Patentinhaber: **BASF Aktiengesellschaft**
**Carl-Bosch-Strasse 38**
**D-67063 Ludwigshafen (DE)**

(72) Erfinder: **Rentzea, Costin, Dr.**
**Richard-Kuhn-Strasse 1-3**
**D-6900 Heidelberg (DE)**
Erfinder: **Rademacher, Wilhelm, Dr.**
**Austrasse 1**
**D-6703 Limburgerhof (DE)**
Erfinder: **Harreus, Albrecht, Dr.**
**Teichgasse 13**
**D-6700 Ludwigshafen (DE)**
Erfinder: **Jung, Johann, Prof. Dr.**
**Hardenburgstrasse 19**
**D-6703 Linburgerhof (DE)**

EP 0 350 693 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Azetidinderivate der allgemeinen Formel I

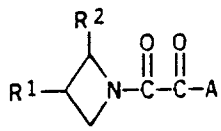

(I)

worin

| | |
|---|---|
| $R^1$ und $R^2$ | für Wasserstoff oder $C_1$-$C_3$-Alkyl und |
| A | für die Gruppe $-XR^3$ oder $-N(R^5)-OR^4$ stehen, worin |
| X | Sauerstoff oder Schwefel, |
| $R^3$ | $C_1$-$C_{20}$-Alkyl, $C_1$-$C_{20}$-Halogenalkyl, $C_3$-$C_{18}$-Alkenyl, $C_3$- oder $C_4$-Alkinyl, ggf. überbrücktes $C_3$-$C_{12}$-Cycloalkyl oder $C_4$-$C_{12}$-Alkyl-cycloalkyl, wobei die Cycloalkylreste bzw. Alkylcycloalkylreste durch $C_1$-$C_5$-Alkyl oder Cyclohexyl substituiert sein können, |
| $R^4$ | $C_1$-$C_{20}$-Alkyl, $C_1$-$C_{12}$-Halogenalkyl, $C_1$-$C_6$-Cyanalkyl, $C_1$-$C_{12}$-Alkoxyalkyl, $C_3$-$C_{18}$-Alkenyl, $C_3$-$C_{18}$-Halogenalkenyl, $C_3$- oder $C_4$-Alkinyl, $C_3$-$C_{12}$-Cycloalkyl oder $C_4$-$C_{12}$-Cycloalkylalkyl, $C_3$-$C_{16}$-Alkoxycarbonylalkyl oder jeweils gegebenenfalls substituiertes $C_7$-$C_{20}$-Aralkyl und |
| $R^5$ | Wasserstoff oder $C_1$-$C_3$-Alkyl bedeuten, |

ausgenommen N-Methoxyoxalylazetidin, sowie Mittel, welche diese Azetidinderivate oder N-Methoxyoxalylazetidin als Wirkstoffe enthalten, Verfahren zu ihrer Herstellung und Verfahren zur Regulierung des Pflanzenwachstums mit diesen Verbindungen oder mit N-Methoxyoxalylazetidin.

N-Methoxyazetidin ist aus der Literatur bekannt (J. Org. Chm. 29 (15), 2264 (1974)). Über eine mögliche Anwendbarkeit dieser Verbindung wurde jedoch nicht berichtet. Es ist weiterhin bekannt, daß bestimmte 2-Halogenethyl-trialkylammonium-halogenide pflanzenwachstumsregulierende Eigenschaften aufweisen (vgl. US-PS 3 156 554). So läßt sich mit Hilfe von (2-Chlorethyl)-trimethylammoniumchlorid das Wachstum von Getreide beeinflussen (vgl. J. Biol. Chem. 235, 475 (1960)). Allerdings ist die Wirksamkeit dieses Stoffes zumindest bei bestimmten Zielorganismen, hinsichtlich Aufwandmenge, Nebenwirkungsarmut und morphospezifischer Wirkung verbesserungsbedürftig.

Der Erfindung lag nun die Aufgabe Zugrunde, neue Verbindungen bereitzusteilen, die eine hohe bioregulatorische, insbesondere das Pflanzenwachstum regulierende Wirksamkeit aufweisen und eine gute Pflanzenverträglichkeit besitzen.

Demgemäß wurden die eingangs definierten Azetidinderivate I sowie Verfahren zu ihrer Herstellung gefunden. Erfindungsgemäß werden weiterhin Mittel, die diese Azetidinderivate oder N-Methoxyoxalylazetidin als Wirkstoff enthalten und ein Verfahren zur Regulierung des Pflanzenwachstums mit diesen Verbindungen sowie mit N-Methoxyoxalylazetidin bereitgestellt.

Im einzelnen haben die Reste $R^1$ bis $R^5$ in Formel I die folgende Bedeutung:

$R^1$ und $R^2$ sind gleich oder verschieden und stehen für Propyl, iso-Propyl, vorzugsweise Ethyl, Methyl und Wasserstoff. Verbindungen, in denen mindestens einer der Reste $R^1$ oder $R^2$ Wasserstoff bedeuten sind bevorzugt.

Der Rest $R^3$ kann breit variiert werden. Beispielsweise steht $R^3$ für geradkettiges oder verzweigtes $C_1$- bis $C_{20}$-Alkyl, insbesondere $C_1$- bis $C_{16}$-Alkyl wie Methyl, Ethyl, n-Propyl, iso-Propyl, n-Butyl, iso-Butyl, sek.-Butyl, Pentyl, Hexyl, Heptyl, Octyl, Nonyl, Decyl, Undecyl, Tridecyl, Tetradecyl, Pentadecyl, Hexadecyl, Heptadecyl, Octadecyl, Nonadecyl; für geradkettiges oder verzweigtes $C_1$-$C_{20}$-Halogenalkyl, insbesondere $C_1$-$C_{12}$-Halogenalkyl mit 1 bis 3, vorzugsweise 1 bis 2 Halogenatomen wie Jod, Brom, insbesondere Fluor oder Chlor wie Chlorbutyl, z.B. 1-Chlorbutyl, 2-Chlorbutyl, 3-Chlorbutyl, 4-Chlorbutyl, 2,3-Dichlorbutyl, Chlorhexyl, z.B. 3-Chlorhexyl, 5-Chlorhexyl, Fluorpentyl, z.B. 3-Fluorpentyl, Fluorhexyl, Fluorheptyl, Fluordecyl, Fluordodecyl; für $C_3$-$C_{18}$-Alkenyl, insbesondere $C_3$-$C_{16}$-Alkenyl mit 1 bis 3 Doppelbindungen im Alkenylrest wie Allyl, Methallyl, Pentenyl, Hexenyl, Heptenyl, Octenyl, Decenyl, Dodecenyl, Geranyl, Hexadecenyl, Octadecenyl; für $C_3$- oder $C_4$-Alkinyl wie Propargyl oder 2-Butinyl; für ggf. überbrücktes, d.h. 1 oder 2 Methylen- oder Ethylenbrücken tragendes $C_3$-$C_{12}$-Cycloalkyl oder $C_4$-$C_{12}$-Alkylcycloalkyl, insbesondere $C_3$-$C_8$-Cycloalkyl bzw. $C_4$-$C_{10}$-Alkylcycloalkyl, wobei die genannten Cycloalkyl- bzw. Alkylcycloalkylreste $C_1$-$C_5$-alkylsubstituiert oder cyclohexylsubstituiert sein können. Beispielsweise seien die folgenden Reste aufgeführt: Cycloalkylreste wie Cyclopropyl, Cyclopentyl, Methylcyclopentyl, Cyclohexyl, Ethylcyclohexyl, Propyl- und Isopropylcyclohexyl, Butyl-, Isobutyl-, sek.-Butyl- und tert. -Butylcyclohexyl, tert.-Amylcyclohexyl, Cyclohexylcyclohexyl, Cycloheptyl, Methylcycloheptyl, Propylcycloheptyl, Cyclooctyl, Cyclododecyl, Decalyl (Decahydronaphthyl); Alkylcycloalkylreste, insbesondere cycloalkylsubstituierte Methylreste wie Cyclopropylmethyl, Cyclopentylmethyl, Cyclohexylmethyl, Cyclododecylmethyl; überbrückte Cycloalkyl- bzw. Alkylcycloalkylreste wie Norbornyl, 1,5-Dimethylbicyclo[2.3.1]octan-8-yl, Tricyclodecanyl, Norbornylmethyl, Adamantyl sowie mono- und bicyclische Terpenreste wie o-Menthyl, m-Menthyl, p-Menthyl, Bornyl, Isobornyl, Pinanyl, Camphenyl und Homocamphenyl.

Der Rest $R^4$ kann ebenfalls breit variiert werden. Beispielsweise steht $R^4$ für

geradkettiges oder verzweigtes $C_1$- bis $C_{20}$-Alkyl, insbesondere $C_1$- bis $C_{16}$-Alkyl wie Methyl, Ethyl, n-Propyl, iso-Propyl, n-Butyl, iso-Butyl, sek.-Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl;

geradkettiges oder $C_1$-$C_{12}$-Halogenalkyl mit 1 bis 3, vorzugsweise 1 bis 2 Halogenatomen wie Jod, Brom, insbesondere Fluor oder Chlor wie Chlorbutyl, z.B. 1-Chlorbutyl, 2-Chlorbutyl, 3-Chlorbutyl, 4-Chlorbutyl, 2,3-Dichlorbutyl, Chlorhexyl, z.B. 3-Chlorhexyl, 5-Chlorhexyl, Fluorpentyl, z.B. 3-Fluorpentyl, Fluorhexyl, Fluorheptyl, Fluordecyl, Fluordodecyl;

$C_1$-$C_6$-Cyanalkyl, insbesondere $C_1$-$C_4$-Cyanalkyl mit einer Cyanogruppe wie z.B. Cyanmethyl, Cyanethyl, Cyanpropyl;

$C_1$-$C_{12}$-Alkoxyalkyl, insbesondere alkoxysubstituiertes $C_1$-$C_8$-Alkyl, wobei als Alkoxyrest insbesondere $C_1$-$C_4$-Alkoxy wie Methoxy, Ethoxy, Propoxy, Isopropoxy, Butoxy, iso-Butoxy, sec.-Butoxy, tert.-Butoxy in Betracht kommen;

$C_3$-$C_{18}$-Alkenyl, insbesondere $C_3$-$C_{16}$-Alkenyl mit 1 bis 3 Doppelbindungen im Alkenylrest wie Allyl, Methallyl, Pentenyl, Hexenyl, Heptenyl, Octenyl, Decenyl, Dodecenyl, Geranyl, Hexadecenyl, Octadecenyl;

$C_3$-$C_{18}$-Halogenalkenyl, insbesondere $C_3$-$C_{12}$-Halogenalkenyl mit 1 bis 3 Doppelbindungen im Alkenylteil und ein oder mehreren, insbesondere 1 bis 3 Halogensubstituenten wie Iod, Brom, insbesondere Fluor oder Chlor. Beispielsweise seien Trifluormethyl, Trichlormethyl, Tribrommethyl, Difluorchlormethyl, Difluormethyl, Pentafluorethyl, Pentachlorethyl, 1,1,2,2-Tetrafluorethyl, 1,2,2-Trifluor-1,2-dichlorethyl, 1-Trifluormethyl-2,2,2-trifluorethyl genannt;

$C_3$- oder $C_4$-Alkinyl wie Propargyl oder 2-Butinyl;

$C_3$-$C_{12}$-Cycloalkyl oder $C_4$-$C_{12}$-Cycloalkylalkyl, insbesondere $C_3$-$C_8$-Cycloalkyl bzw. $C_4$-$C_{10}$-Cycloalkylalkyl, wobei die genannten Cycloalkyl- bzw. Cycloalkylalkylreste $C_1$-$C_5$-alkylsubstituiert oder cyclohexylsubstituiert sein können. Beispielsweise seien die folgenden Reste aufgefuhrt: Cycloalkylreste wie Cyclopropyl, Cyclopentyl, Methylcyclopentyl, Cyclohexyl, Ethylcyclohexyl, Propyl- und Isopropylcyclohexyl, Butyl-, Isobutyl-, sek.-Butyl- und tert.-Butylcyclohexyl, tert.-Amylcyclohexyl, Cyclohexylcyclohexyl, Cycloheptyl, Methylcycloheptyl, Propylcycloheptyl, Cyclooctyl, Cyclododecyl, Di-, Tri- und Tetramethylcyclohexyl; Cycloalkylalkylreste, insbesondere cycloalkylsubstituierte Methylreste wie Cyclopropylmethyl, Cyclopentylmethyl, Cyclohexylmethyl, Cycloheptylmethyl, Cyclododecylmethyl, Cyclohexylethyl, Cyclohexylpropyl; $C_3$-$C_{16}$-Alkoxycarbonylalkyl, z.B. $C_1$-$C_4$-Alkoxycarbonyl-$C_1$-$C_4$-alkyl, wobei

$C_1$-$C_4$-Alkyl im Alkoxy- bzw. Alkylteil für Methyl, Ethyl, Propyl, iso-Propyl, Butyl oder iso-Butyl steht;

$C_7$-$C_{20}$-Aralkyl, insbesondere $C_7$-$C_{12}$-Phenylalkyl wie Benzyl, 2-Phenylethyl, 3-Phenylpropyl, 2-Phenylpropyl, wobei die aromatischen Reste ggf. substituiert sind durch ein oder mehrere, insbesondere 1 bis 3 Substituenten wie Halogen, insbesondere Fluor, Chlor oder Brom, Nitro, Halogenalkyl, z.B. Fluor- oder Chlor-$C_1$-$C_4$-alkyl, wie Trifluormethyl, $C_1$-$C_4$-Alkyl oder -Alkoxy wie Methyl, Ethyl, Propyl, iso-Propyl, n-Butyl, iso-Butyl, sec.-Butyl, tert.-Butyl oder die entsprechenden Alkoxyreste. Beispielsweise seien 4-Fluorbenzyl, 4-Chlorbenzyl, 4-Brombenzyl, 2,4-Dichlorbenzyl, 2-Methylbenzyl, 3-Methylbenzyl, 4-Methylbenzyl, 3-Methoxybenzyl, 4-Methoxybenzyl, 4-Nitrobenzyl, 3-Trifluormethylbenzyl, 4-Trifluormethylbenzyl, 4-tert.-Butylbenzyl und 2,4,6-Trimethylbenzyl genannt.

Die neuen Verbindungen lassen sich herstellen, indem man im Fall von A = $XR^3$

a) Azetidine der Formel II

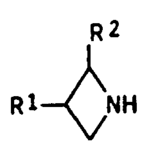

(II)

mit einem Oxalesterchlorid der Formel III

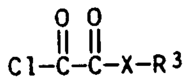

(III),

wobei $R^1$, $R^2$, X und $R^3$ die oben genannte Bedeutung haben, oder
b) ein Oxalamidchlorid der Formel IV

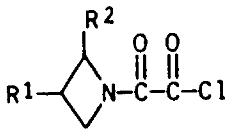

(IV)

mit einem Alkohol oder Thiol der Formel V

$HX-R^3$ (V),

wobei $R^1$, $R^2$, X und $R^3$ die oben angegebenen Bedeutungen haben, gegebenenfalls in Gegenwart eines Lösungs- oder Verdünnungsmittels und/oder einer anorganischen oder organischen Base und/oder eines Reaktionsbeschleunigers umsetzt.

Im Fall von A = $NR^5$-$OR^4$ lassen sich die neuen Verbindungen herstellen, indem man
a) Oxalamidchloride der Formel IV

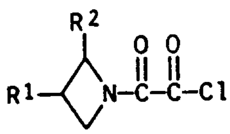

(IV)

mit einem Hydroxylamin-Derivat der Formel VI

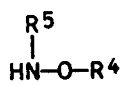

(VI)

wobei $R^1$, $R^2$, $R^4$ und $R^5$ die oben angegebene Bedeutung haben, oder
b) ein Oxalamidester der Formel VII

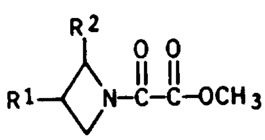

(VII)

mit einem Hydroxylamin-Derivat der Formel VI

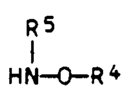

(VI)

wobei $R^1$, $R^2$, $R^4$ und $R^5$ die oben angegebenen Bedeutungen haben, gegebenenfalls in Gegenwart eines Lösungs- oder Verdünnungsmittels und/oder einer anorganischen oder organischen Base und/oder eines Reaktionsbeschleunigers umsetzt.

Für beide Verfahrensvarianten a) und b) im Fall von A = $XR^3$ oder $NR^5$-$OR^4$ kommen als Lösungs- oder Verdünnungsmittel beispielsweise Halogenkohlenwasserstoffe, insbesondere Chlorkohlenwasserstoffe,

z.B. Tetrachlorethylen-1,1,2,2- oder 2,1,1,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1,2-Trichlorethan, Trichlorethylen, Pentachlorethan, o-, m-, p-Difluorbenzol, 1,2-Dichlorethan, 1,1-Dichlorethan, 1,2-cis-Dichlorethylen, Chlorbenzol, Fluorbenzol, Brombenzol, Jodbenzol, o-, m-, p-Dichlorbenzol, o-, p-, m-Dibrombenzol, o-, m-, p-Chlortoluol, 1,2,4-Trichlorbenzol; Ether, z.B. Ethylpropylether, Methyl-tert.-butylether, n-Butylethylether, Di-n-butylether, Di-isobutylether, Diisoamylether, Diisopropylether, Anisol, Phenetol, Cyclohexylmethylether, Diethylether, Ethylenglykoldimethylether, Tetrahydrofuran, Dioxan, Thioanisol, Nitrokohlenwasserstoffe wie Nitromethan, Nitroethan, Nitrobenzol, o-, m-, p-Chlornitrobenzol, o-Nitrotoluol; Nitrile wie Acetonitril, Butyronitril, Isobutyronitril, Benzonitril, m-Chlorbenzonitril; aliphatische oder cycloaliphatische Kohlenwasserstoffe, z.B. Heptan, Pinan, Nonan, o-, m-, p-Cymol, Benzinfraktionen innerhalb eines Siedepunktintervalles von 70 bis 190 °C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan; Ester z.B. Ethylacetat, Acetessigester, Isobutylacetat; Amide, z.B. Formamid, Methylformamid, Dimethylformamid; Ketone, z.B. Aceton, Methylethylketon, gegebenenfalls auch Wasser und entsprechende Gemische in Betracht. Als Lösungsmittel können auch die Verbindungen der Formeln III bis V bzw. IV, VI und VII im Überschuß verwendet werden. Zweckmäßig verwendet man das Lösungsmittel in einer Menge von 100 bis 2 000 Gew.-%, vorzugsweise von 200 bis 700 Gew.-%, bezogen auf Ausgangsstoff II im Fall von A = $XR^3$ oder bezogen auf den Ausgangsstoff IV oder VII im Fall von A = $NR^5$-$OR^4$.

Als anorganische oder organische Basen (Säureakzeptoren) für die Umsetzung zu Verbindungen der Formel I können alle üblichen Säurebindemittel verwendet werden. Hierzu gehören vorzugsweise tertiäre Amine, Erdalkaliverbindungen, Ammoniumverbindungen und Alkaliverbindungen sowie entsprechende Gemische.

Es kommen z.B. als basische Verbindungen in Frage: Kaliumhydroxid, Natriumhydroxid, Kaliumcarbonat, Natriumcarbonat, Lithiumhydroxid, Lithiumcarbonat, Natriumbicarbonat, Kaliumbicarbonat, Calciumhydroxid, Calciumoxid, Bariumoxid, Magnesiunmhydroxid, Magnesiumoxid, Bariumhydroxid, Calciumcarbonat, Magnesiumcarbonat, Magnesiumbicarbonat, Magnesiumacetat, Zinkhydroxid₁ Zinkoxid, Zinkcarbonat, Zinkbicarbonat, Zinkacetat, Natriumformiat, Natriumacetat, Trimethylamin, Triethylamin, Tripropylamin, Triisopropylamin₁ Tributylamin, Triisobutylamin, Tri-sec.-butylamin, Tribenzylamin, Tricyclohexylamin, Triamylamin, Trihexylamin, N,N-Dimethylanilin, N,N-Diethylanilin, N,N-Dipropylanilin, N,N-Dimethyltoluidin, N,N-Diethyltoluidin, N,N-Dipropyltoluidin, N,N-Dimethyl-p-aminopyridin, N,N-Diethyl-p-aminopyridin, N,N-Dipropylp-aminopyridin, N-Methylpyrrolidin, N-Ethylpyrrolidin, N-Methylpiperidin, N-Ethylpiperidin, N-Methylimidazol, N-Ethylimidazol, N-Methylmorpholin, N-Ethylmorpholin, N-Methylhexamethylenimin, N-Ethylhexamethylenimin, Pyridin, Chinolin, alpha-Picolin, beta-Picolin, gamma-Picolin, Isochinolin, Pyrimidin, Acridin, N,N,N',N'-Tetramethylethylendiamin, N,N,N',N'-Tetraethylethylendiamin, Chinoxalin, Chinazolin, N-Propyldiisopropylamin, N,N-Dimethylcyclohexylamin, 2,6-Lutidin, 2,4-Lutidin, Trifurfurylamin, Triethylendiamin.

Zweckmäßig verwendet man den Säureakzeptor in stöchiometrischen Mengen, im Überschuß oder Unterschuß von jeweils bis zu 20 Mol.%, bezogen auf den Ausgangsstoff III im Fall von A = $XR^3$ bzw. bezogen auf den Ausgangsstoff IV im Fall von A = $NR^5$-$OR^4$.

Als Reaktionsbeschleuniger kommen vorzugsweise Metallhalogenide, insbesondere Alkalimetallhalogenide wie Natriumiodid oder Kaliumiodid in Frage.

Die Herstellung der erfindungsgemäßen Wirkstoffe wird durch folgende Beispiele erläutert:

Beispiel 1.1

Azetidinoxamid-butylester

200 ml Methylenchlorid werden zwischen 10 und 20 °C nacheinander mit 15,5 g (0,15 mol) Triethylamin, 8,6 g (0,15 mol) Azetidin und 24,7 g (0,15 mol) Oxalsäurebutylesterchlorid versetzt. Nach zehnstündigem Nachrühren bei 25 °C wird das Gemisch zweimal mit Wasser gewaschen, über $Na_2SO_4$ getrocknet und anschließend fraktioniert destilliert. Man erhält 18,8 g (67,7 % der Theorie) Azetidinoxamid-butylester als farblose Flüssigkeit vom Sdp. 122-123 °C/0,45 mbar und $n_D^{21}$ 1,4702.

Entsprechend können durch Wahl der Ausgangsstoffe und entsprechende Anpassung der Verfahrensbedingungen die nachstehend aufgelisteten Verbindungen der Formel I erhalten werden:

Tabelle 1: Verbindungen der allgemeinen Formel I mit A = $XR^3$

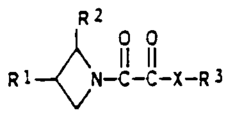

(I),

| Nr. | $R^1$ | $R^2$ | X | $R^3$ | Brechungsindex, Fp (°C) oder Sdp. (°C/mbar) |
|---|---|---|---|---|---|
| 1.1 | H | H | O | $-C_4H_9$ | 122-123/0,45 |
| 1.2 | H | H | O | $-CH_3$ | 96- 98/0,4 *) |
| 1.3 | H | H | S | $-C_2H_5$ | $n^{22}_D$ 1,5340 |
| 1.4 | H | H | O | $-C_2H_5$ | 98-101/0,1 |
| 1.5 | $CH_3$ | H | O | $-C_2H_5$ | 129-131/0,3 |
| 1.6 | $C_2H_5$ | H | O | $-C_2H_5$ | 136-140/0,2 |
| 1.7 | H | H | O | $-CH_2-CH_2-Cl$ | $n^{23}_D$ 1,4999 |
| 1.8 | H | H | O | $-n-C_3H_7$ | 106-110/0,12 |
| 1.9 | H | $CH_3$ | O | $-n-C_3H_7$ | 128-131/0,3 |
| 1.10 | H | H | S | $-n-C_3H_7$ | $n^{22}_D$ 1,5266 |
| 1.11 | H | H | O | $-CH_2-CH=CH_2$ | 106-110/0,3 |
| 1.12 | H | H | S | $-CH_2-CH=CH_2$ | 116-118/0,15 |
| 1.13 | H | H | O | | Fp. 67- 69 |
| 1.14 | H | H | S | $-n-C_4H_9$ | $n^{22}_D$ 1,5200 |
| 1.15 | $CH_3$ | H | O | $-n-C_4H_9$ | 129-132/0,2 |
| 1.16 | H | H | O | $-i-C_4H_9$ | 106-110/0,1 |
| 1.17 | H | H | O | sek.-$C_4H_9$ | 112-115/0,15 |
| 1.18 | H | H | O | tert.-$C_4H_9$ | 98-105/0,1 |
| 1.19 | H | H | O | $-CH_2-C(CH_3)=CH_2$ | 111-114/0,3 |
| 1.20 | H | H | O | $-CH_2-CH=CH-CH_3$(trans) | 123-124/0,3 |
| 1.21 | H | H | O | $n-C_5H_{11}$ | 117-119/0,2 |
| 1.22 | H | H | O | $-CH_2-CH_2-CH(CH_3)_2$ | 125-130/0,3 |
| 1.23 | H | H | O | -Cyclopentyl | 112-117/0,2 |
| 1.24 | H | H | O | $-CH_2-CH=C(CH_3)_2$ | 127-130/0,3 |
| 1.25 | H | H | O | $-CH_2-CH=CH-CH_2-CH_3$(trans) | 122-128/0,15 |
| 1.26 | H | H | O | $n-C_6H_{13}$ | 120-122/0,1 |
| 1.27 | H | H | O | -Cyclopentylmethyl | 129-132/0,2 |
| 1.28 | H | H | O | -Cyclohexyl | 125-128/0,15 |
| 1.29 | H | H | O | $-C_6H_{13}$ | 132-135/0,15 |
| 1.30 | H | H | O | $-(CH_2)_6-Cl$ | 127-129/0,2 |
| 1.31 | H | H | O | -Cyclohexylmethyl | 138-139/0,15 |
| 1.32 | H | H | O | -Cycloheptyl | 132-135/0,1 |
| 1.34 | H | H | O | $n-C_8H_{17}$ | $n^{22}_D$ 1,4682 |
| 1.35 | H | H | O | $-CH_2-CH(C_2H_5)-(CH_2)_3-CH_3$ | 152-157/0,25 |

*) Sdp. 97,5 bis 98,5°C/1,2 mm: Sheehan et al., J. Org. Chem. 39 (15), 2264 (1974). (Als Verbindung nicht beansprucht),

Tabelle 1 - Forts.

| Nr. | $R^1$ | $R^2$ | X | $R^3$ | Brechungsindex, Fp (°C) oder Sdp. (°C/mbar) |
|---|---|---|---|---|---|
| 1.36 | H | H | O | -Geranyl | Öl |
| 1.37 | H | H | O | -Cyclohexylethyl | 144-147/0,2 |
| 1.38 | H | H | O | -Menthyl | 131-135/0,1 |
| 1.39 | H | H | O | -Norbornyl | 128-129/0,1 |
| 1.40 | H | H | O | -Pinanyl | 131-135/0,3 |
| 1.41 | H | H | O | -4-tert.-Butylcyclohexyl | 149-152/0,3 |
| 1.42 | H | H | O | n-$C_9$-$H_{19}$ | $n_D^{28}$ 1,4686 |
| 1.43 | H | H | O | n-$C_{10}H_{21}$ | Fp. 36-38 |
| 1.44 | H | H | O | -9-Decen-1-yl | Fp. 30-32 |
| 1.45 | H | H | O | n-$C_{12}H_{25}$ | Fp. 47-48 |
| 1.46 | H | H | O | n-$C_{14}H_{29}$ | Fp. 55-56 |
| 1.47 | H | H | O | n-$C_{16}H_{33}$ | Fp. 61-62 |

Beispiel 2.1

O-(2-Methyl-2-propen-1-yl)-azetidinoxamid-hydroxamsäure

100 ml Methanol wurden bei 25 °C nacheinander mit 14,3 g (0,1 mol) Azetidinoxamid-methylester, 18,5 g (0,15 Mol) O-(2-Methyl-2-propen-1-yl)-hydroxylamin-hydrochlorid und 15 g (0,15 Mol) Triethylamin versetzt. Das Gemisch wurde 10 Stunden bei 25 °C und weitere 10 Stunden bei 70 °C nachgerührt und anschließend eingeengt. Der Rückstand wurde mit 200 ml Methylenchlorid versetzt und dreimal mit je 50 ml Wasser gewaschen, über $Na_2SO_4$ getrocknet und im Vakuum eingeengt. Der Rückstand wurde mit 20 ml Ether versetzt und die Kristalle abgesaugt und getrocknet. Man erhielt 12,4 g (62,6 % d. Th.) O-(2-Methyl-2-propen-1-yl)-azetindioxamid-hydroxamsäure als weiße Kristalle vom Schmp. 92-94 °C.

Entsprechend können durch Wahl der Ausgangsstoffe und entsprechende Anpassung der Verfahrensbedingungen die nachstehend aufgelisteten Verbindungen der Formel I erhalten werden:

Tabelle 2: Verbindungen der allgemeinen Formel I mit A = $-N(R^5)-OR^4$

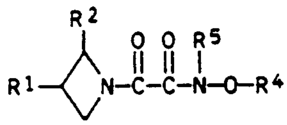

(I)

| Beispiel Nr. | $R^1$ | $R^2$ | $R^5$ | $R^4$ | Fp (°C) oder Sdp. (°C/mbar) |
|---|---|---|---|---|---|
| 2.2 | H | H | H | H | 149-151 |
| 2.3 | H | $CH_3$ | H | H | 91- 92 |
| 2.4 | H | H | H | $CH_3$ | 103-105 |
| 2.5 | H | H | H | $C_2H_5$ | 73- 77 |
| 2.6 | H | H | H | $C_3H_7$-n | 66- 67 |
| 2.7 | H | $CH_3$ | H | $C_3H_7$-n | 137-140/0,3 |
| 2.8 | $CH_3$ | $CH_3$ | H | $C_3H_7$-n | 141-145/0,2 |
| 2.9 | H | H | $CH_3$ | $CH_3$ | 97-100 |
| 2.10 | H | H | H | $CH_2-CH=CH_2$ | 114-117 |
| 2.11 | H | H | H | $CH_2-CH=CH-CH_3$ | 138-140 |
| 2.12 | H | H | H | $CH_2-CH=CH-Cl$ | 145-148 |
| 2.13 | H | H | H | $CH_2-C(Cl)=CH_2$ | 93- 96 |
| 2.14 | H | H | H | $CH_2-C{\equiv}CH$ | 120-123 |
| 2.15 | H | H | H | $C_4H_9$-n | 62- 64 |
| 2.16 | H | $CH_3$ | H | $C_4H_9$-n | 142-153/0,2 |
| 2.17 | H | H | H | $C_5H_{11}$-n | 80- 83 |
| 2.18 | H | H | H | $C_6H_{13}$-n | 89- 92 |
| 2.19 | H | H | H | $(CH_2)_3-Cl$ | 59- 62 |
| 2.20 | H | H | H | $(CH_2)_4-Cl$ | 75- 78 |
| 2.21 | H | H | H | $CH_2-C_6H_{11}$ | 135-137 |
| 2.22 | H | H | H | $CH_2-C_6H_5$ | 160-163 |
| 2.23 | H | H | H | $2-Cl-C_6H_4-CH_2$ | Harz |
| 2.24 | H | H | H | $4-Cl-C_6H_4-CH_2$ | 179-182 |

Die Verbindungen der Formel I können praktisch alle Entwicklungstadien einer Pflanze verschiedenartig beeinflussen und werden deshalb als Wachstums- oder Bioregulatoren eingesetzt. Die Wirkungsvielfalt der Pflanzenwachstumsregulatoren hängt ab vor allem

a) von der Pflanzenart und -sorte,

b) von dem Zeitpunkt der Applikation, bezogen auf das Entwicklungsstadium der Pflanze und von der Jahreszeit,

c) von dem Applikationsort und -verfahren (Samenbeize, Bodenbehandlung oder Blattapplikation)

d) von klimatischen Faktoren, z.B. Temperatur, Niederschlagsmenge, außerdem auch Tageslänge und Lichtintensität

e) von der Bodenbeschaffenheit (einschließlich Düngung),

f) von der Formulierung bzw. Anwendungsform des Wirkstoffs und schließlich

g) von den angewendeten Konzentrationen der aktiven Substanz.

Aus der Reihe der verschiedenartigen Anwendungsmöglichkeiten der erfindungsgemäßen Pflanzenwachstumsregulatoren im Pflanzenanbau, werden einige nachstehend erwähnt.

A.

Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist sich z.B. die Verringerung des Grasbewuchses an Straßenrändern, Hecken, Kanalböschungen und auf Rasenflächen wie Park-, Sport- und Obstanlagen, zierrasen und Flugplätzen, so daß der arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Reis, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit Bioregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt.

Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- und Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen die dichtere Bepflanzung des Bodens, so daß ein Mehrertrag, bezogen auf die Bodenfläche, erzielt werden kann.

B.

Mit den neuen Mitteln lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Dabei können die Verbindungen der Formel I Ertragssteigerungen durch Eingriffe in den pflanzlichen Stoffwechsel bzw. durch Förderung oder Hemmung des vegetativen und/oder des generativen Wachstums verursachen.

C.

Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen. Von wirtschaftichem Interesse ist beispielsweise die Ernteerleicherung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt- und Sproßteil der Pflanze ist auch für ein gut kontrollierbares Entblättern von Nutzpflanzen wesentlich.

D.

Mit Wachstumsregulatoren kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.

- die Öffnungsweite des Stomata reduziert wird
- eine dickere Epidermis und Cuticula ausgebildet werden
- die Durchwurzelung des Bodens verbessert wird
- das Mikroklima im Pflanzenbestand durch einen kompakteren Wuchs günstig beeinflußt wird.

Die erfindungsgemäß zu verwendeten Wirkstoffe können den Kulturpflanzen sowohl vom Samen her (als Saatgutbeizmittel) als auch über den Boden, d.h. durch die Wurzel sowie über das Blatt zugeführt werden.

Infolge der hohen Pflanzenverträglichkeit kann die Aufwandmenge stark variiert werden.

Bei der Saatgutbehandlung werden im allgemeinen Wirkstoffmengen von 0,001 bis 50 g, vorzugsweise 0,01 bis 10 g je Kilogramm Saatgut benötigt.

Für die Blatt- und Bodenbehandlung sind im allgemeinen Gaben von 0,01 bis 10 kg/ha, bevorzugt 0,05 bis 3 kg/ha, als ausreichend zu betrachten.

Die erfindungsgemäßen Mittel können in Form üblicher Formulierungen angewendet werden wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Amine (z.B. Ethanolamin), N,N-Dimethylformamid und Wasser; feste Trägerstoffe wie natürliche Gesteinsmehle (z.B. Kaoline, Tonerden, Talkum, Kreide) und synthetische Gesteinsmehle (z.B. hochdisperse Kieselsäure, Silikate) ; Emulgiermittel oder sonstige oberflächenaktive Mittel, wie nichtionogene und anionische Emulgatoren (z.B. Polyoxyethylen-Fettalkohol-Ether, Alkylsulfonate) und Dispergiermittel wie Lignin, Sulfitablaugen und Methylcellulose. Bevorzugt ist die Anwendung der erfindungsgemäßen Verbindungen in wäßriger Lösung gegebenenfalls unter Zusatz von mit Wasser mischbaren organischen Lösungsmitteln wie Methanol oder anderen niederen Alkoholen, Aceton, N,N-Dimethylformamid oder N-Methylpyrrolidon. Die Formulierungen enthalten im allgemeinen zwischen 0,1 und 95 Gew.% Wirkstoff, vorzugsweise zwischen 0,5 und 90 Gew.%.

Die Formulierungen bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise im Vorauflaufverfahren, im Nachauflaufverfahren oder als Beizmittel.

Beispiele für Formulierungen sind:

I. 20 Gewichtsteile der Verbindung des Beispiel 1.1 werden in 3 Gewichtsteilen des Natriumsalzes der Diisobutylnaphthalinsulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

II. 3 Gewichtsteile der Verbindung des Beispiels 1.13 werden mit 97 Gewichtsteilen feinteiligem Kaolin innig vermischt. Man erhält auf diese Weise ein Stäubemittel, das 3 Gew.% des Wirkstoffs enthält.

III. 30 Gewichtsteile der Verbindung des Beispiels 1.11 werden in einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

IV. 20 Teile der Verbindung des Beispiels 1.10 werden mit 2 Teilen Calciumsalz der Dodecylbenzolsulfonsäure, 8 Teilen Fettalkoholpolyglykolether, 2 Teilen Natriumsalz eines Phenolsulfonsäureharnstoffformaldehyd-Kondensats und 68 Teilen eines paraffinischen Mineralöls innig vermischt. Man erhält eine stabile ölige Dispersion.

V. Man vermischt 90 Gewichtsteile der Verbindung des Beispiels 2.1 mit 10 Gewichtsteilen N-Methyl-α-pyrrolidon und erhält eine Lösung, die zur Anwendung in Form kleinster Tropfen geeignet ist.

VI. 20 Gewichtsteile der Verbindung des Beispiels 2.10 werden in einer Mischung gelöst, die aus 80 Gewichtsteilen Xylol 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

VII. 20 Gewichtsteile der Verbindung des Beispiels 1.25 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

VIII. 20 Gewichtsteile der Verbindung des Beispiels 2.12 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

Die erfindungsgemäßen Mittel können in diesen Anwendungsformen auch zusammen mit anderen Wirkstoffen vorliegen, wie z.B. Herbiziden, Insektiziden, oder auch mit Düngemitteln vermischt und ausgebracht werden. Beim Vermischen mit anderen Wachstumsregulatoren treten auch synergistische Effekte auf, d.h. die Wirksamkeit des Kombinationsproduktes ist größer als die addierten Wirksamkeiten der Einzelkomponenten.

Anwendungsbeispiele

Zur Bestimmung der wachstumsregulierenden Eigenschaften der Prüfsubstanzen wurden die Testpflanzen auf ausreichend mit Nährstoffen versorgtem Kultursubstrat in Kunststoffgefäßen (ca. 12,5 cm Durchmesser; Volumen ca. 500 ml) angezogen.

Im Nachauflaufverfahren wurden die zu prüfenden Substanzen in wäßriger Aufbereitung auf die Pflanzen gesprüht. Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt. Als Vergleichssubstanz A diente 2-Chlorethyltrimethylammoniumchlorid (CCC).

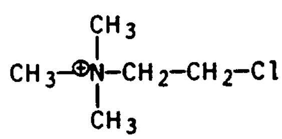

(A)

Gleichlaufend zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

Die Ergebnisse sind den folgenden Tabellen zu entnehmen:

Tabelle 3
Sommerweizen, Sorte "Kolibri" - Nachauflauf-Blattbehandlung

| Nr. d. chem. Beispiele | Wirkstoffkonz. pro Gefäß [mg] | relative Wuchshöhen |
|---|---|---|
| unbehandelt | - | 100 |
| "A" | 0,38 | 95,2 |
| | 1,5 | 90,3 |
| 1.1 | 0,38 | 91,9 |
| | 1,5 | 72,5 |
| 1.2 | 0,38 | 77,2 |
| | 1,5 | 49,6 |
| 1.3 | 0,38 | 78,7 |
| | 1,5 | 53,9 |
| 1.7 | 0,38 | 84,1 |
| | 1,5 | 60,8 |
| 1.8 | 0,38 | 90,6 |
| | 1,5 | 79,0 |
| 1.10 | 0,38 | 78,7 |
| | 1,5 | 52,5 |
| 1.11 | 0,38 | 82,9 |
| | 1,5 | 64,7 |
| 1.12 | 0,38 | 75,8 |
| | 1,5 | 49,6 |
| 1.13 | 0,38 | 84,1 |
| | 1,5 | 63,4 |
| 1.14 | 0,38 | 75,8 |
| | 1,5 | 43,7 |
| 1.16 | 0,38 | 90,6 |
| | 1,5 | 80,3 |
| 1.19 | 0,38 | 85,4 |
| | 1,5 | 68,6 |
| 1.20 | 0,38 | 85,4 |
| | 1,5 | 64,7 |
| 1.21 | 0,38 | 89,3 |
| | 1,5 | 66,0 |
| 1.24 | 0,38 | 88,0 |
| | 1,5 | 64,7 |
| 1.26 | 0,38 | 84,1 |
| | 1,5 | 63,4 |
| 1.27 | 0,38 | 83,9 |
| | 1,5 | 72,6 |

Tabelle 3 (Fortsetzung)
Sommerweizen, Sorte "Kolibri" - Nachauflauf-Blattbehandlung

| Nr. d. chem. Beispiele | Wirkstoffkonz. pro Gefäß [mg] | relative Wuchshöhen |
|---|---|---|
| 1.34 | 0,38 | 82,3 |
| | 1,5 | 66,1 |
| 1.36 | 0,38 | 88,0 |
| | 1,5 | 69,9 |
| 1.42 | 0,38 | 79,0 |
| | 1,5 | 64,5 |
| 1.43 | 0,38 | 82,3 |
| | 1,5 | 69,4 |
| 1.45 | 0,38 | 85,5 |
| | 1,5 | 75,8 |
| 1.46 | 0,38 | 93,6 |
| | 1,5 | 88,7 |

Tabelle 4

Sommergerste, Sorte "Aramir" - Nachauflauf-Blattbehandlung

| Nr. d. chem. Beispiele | Wirkstoffkonz. pro Gefäß [mg] | relative Wuchshöhen |
|---|---|---|
| unbehandelt | - | 100 |
| "A" | 0,38 | 94,5 |
| | 1,5 | 94,5 |
| 1.1 | 0,38 | 89,0 |
| | 1,5 | 60,6 |
| 1.2 | 0,38 | 87,6 |
| | 1,5 | 65,0 |
| 1.3 | 0,38 | 87,6 |
| | 1,5 | 66,4 |
| 1.7 | 0,38 | 87,8 |
| | 1,5 | 56,9 |
| 1.8 | 0,38 | 89,0 |
| | 1,5 | 64,3 |
| 1.10 | 0,38 | 90,5 |
| | 1,5 | 67,9 |
| 1.11 | 0,38 | 80,4 |
| | 1,5 | 54,4 |
| 1.12 | 0,38 | 87,6 |
| | 1,5 | 60,8 |
| 1.13 | 0,38 | 85,3 |
| | 1,5 | 59,4 |
| 1.14 | 0,38 | 84,8 |
| | 1,5 | 53,7 |
| 1.16 | 0,38 | 90,3 |
| | 1,5 | 59,4 |
| 1.19 | 0,38 | 87,8 |
| | 1,5 | 51,9 |
| 1.20 | 0,38 | 86,6 |
| | 1,5 | 51,9 |
| 1.21 | 0,38 | 84,1 |
| | 1,5 | 49,5 |
| 1.24 | 0,38 | 85,3 |
| | 1,5 | 59,4 |
| 1.26 | 0,38 | 85,3 |
| | 1,5 | 53,2 |

Tabelle 4 - Forts.

Sommergerste, Sorte "Aramir" - Nachauflauf-Blattbehandlung

| Nr. d. chem. Beispiele | Wirkstoffkonz. pro Gefäß [mg] | relative Wuchshöhen |
|---|---|---|
| 1.27 | 0,38<br>1,5 | 85,0<br>50,4 |
| 1.34 | 0,38<br>1,5 | 85,0<br>50,4 |
| 1.36 | 0,38<br>1,5 | 87,8<br>58,1 |
| 1.42 | 0,38<br>1,5 | 80,3<br>53,5 |
| 1.43 | 0,38<br>1,5 | 81,9<br>53,5 |
| 1.45 | 0,38<br>1,5 | 80,3<br>59,8 |
| 1.46 | 0,38<br>1,5 | 86,6<br>69,3 |

Tabelle 5

| Sommerweizen, Sorte "Kolibri" - Nachauflauf-Blattbehandlung | | |
|---|---|---|
| Nr. d. chem. Beispiele | Wirkstoffkonz. pro Gefäß [mg] | relative Wuchshöhen |
| unbehandelt | - | 100 |
| "A" | 1,5<br>6 | 90,3<br>87,1 |
| 1.4 | 1,5<br>6 | 66,5<br>48,7 |

Tabelle 6

| Sommergerste, Sorte "Aramir" - Nachauflauf-Blattbehandlung | | |
|---|---|---|
| Nr. d. chem. Beispiele | Wirkstoffkonz. pro Gefäß [mg] | relative Wuchshöhen |
| unbehandelt | - | 100 |
| "A" | 1,5<br>6 | 94,5<br>91,3 |
| 1.4 | 1,5<br>6 | 75,1<br>40,2 |

Tabelle 7

| Reis, Sorte "Bahia" - Nachauflauf-Blattbehandlung | | |
|---|---|---|
| Nr. d. chem. Beispiele | Wirkstoffkonz. pro Gefäß [mg] | relative Wuchshöhen |
| unbehandelt | - | 100 |
| "A" | 1,5<br>3 | 98,6<br>98,6 |
| 1.1 | 1,5<br>3 | 73,2<br>70,4 |
| 1.7 | 1,5<br>3 | 73,2<br>67,6 |
| 1.8 | 1,5<br>3 | 76,1<br>73,2 |
| 1.11 | 1,5<br>3 | 70,4<br>64,8 |
| 1.13 | 1,5<br>3 | 67,6<br>64,8 |
| 1.16 | 1,5<br>3 | 76,1<br>70,4 |
| 1.19 | 1,5<br>3 | 70,4<br>67,6 |
| 1.20 | 1,5<br>3 | 71,8<br>66,2 |
| 1.21 | 1,5<br>3 | 71,8<br>67,6 |
| 1.24 | 1,5<br>3 | 73,2<br>69,0 |
| 1.26 | 1,5<br>3 | 70,4<br>63,4 |
| 1.27 | 1,5<br>3 | 71,8<br>67,6 |
| 1.36 | 1,5<br>3 | 71,8<br>67,6 |

Tabelle 8

| Reis, Sorte "Bahia" - Nachauflauf-Blattbehandlung | | |
|---|---|---|
| Nr. d. chem. Beispiele | Wirkstoffkonz. pro Gefäß [mg] | relative Wuchshöhen |
| unbehandelt | - | 100 |
| "A" | 1,5<br>6 | 99,8<br>97,1 |
| 1.2 | 1,5<br>6 | 80,1<br>53,8 |
| 1.3 | 1,5<br>6 | 81,4<br>52,5 |
| 1.10 | 1,5<br>6 | 88,0<br>52,5 |
| 1.12 | 1,5<br>6 | 85,3<br>53,8 |
| 1.14 | 1,5<br>6 | 88,0<br>55,1 |

Tabelle 9

| Sonnenblumen, Sorte "Sorex" - Nachauflauf-Blattbehandlung | | |
|---|---|---|
| Nr. d. chem. Beispiele | Wirkstoffkonz. pro Gefäß [mg] | relative Wuchshöhen |
| unbehandelt | - | 100 |
| "A" | 6 | 85,6 |
| 1.4 | 6 | 58,2 |
| 1.7 | 6 | 47,6 |
| 1.19 | 6 | 50,7 |
| 1.24 | 6 | 50,7 |

Tabelle 10

| Sommerraps, Sorte "Petranova" - Nachauflauf-Blattbehandlung | | |
|---|---|---|
| Nr. d. chem. Beispiele | Wirkstoffkonz. pro Gefäß [mg] | relative Wuchshöhen |
| unbehandelt | - | 100 |
| "A" | 1,5<br>6 | 94,1<br>94,1 |
| 1.2 | 1,5<br>6 | 68,6<br>68,6 |
| 1.3 | 1,5<br>6 | 70,6<br>70,6 |
| 1.10 | 1,5<br>6 | 70,6<br>70,6 |
| 1.12 | 1,5<br>6 | 70,6<br>66,7 |
| 1.14 | 1,5<br>6 | 70,6<br>70,6 |

Tabelle 11

| Baumwolle, Sorte "Delta Pine" - Nachauflauf-Blattbehandlung | | |
|---|---|---|
| Nr. d. chem. Beispiele | Wirkstoffkonz. pro Gefäß [mg] | relative Wuchshöhen |
| unbehandelt | - | 100 |
| "A" | 0,5 | 80,1 |
| 1.1 | 0,5 | 71,0 |
| 1.8 | 0,5 | 74,4 |
| 1.11 | 0,5 | 69,8 |
| 1.13 | 0,5 | 64,1 |
| 1.16 | 0,5 | 73,3 |
| 1.19 | 0,5 | 68,7 |
| 1.20 | 0,5 | 75,5 |
| 1.21 | 0,5 | 68,7 |
| 1.24 | 0,5 | 72,1 |
| 1.26 | 0,5 | 74,4 |
| 1.27 | 0,5 | 73,3 |
| 1.36 | 0,5 | 73,3 |

Tabelle 12

| Mais, Sorte "Dea" - Nachauflauf-Blattbehandlung | | |
|---|---|---|
| Nr. d. chem. Beispiele | Wirkstoffkonz. pro Gefäß [mg] | relative Wuchshöhen |
| unbehandelt | - | 100 |
| "A" | 1,5<br>6 | 101,1<br>101,1 |
| 1.2 | 1,5<br>6 | 64,9<br>63,4 |
| 1.3 | 1,5<br>6 | 69,4<br>63,4 |
| 1.4 | 1,5<br>6 | 66,4<br>63,4 |
| 1.10 | 1,5<br>6 | 72,5<br>69,4 |
| 1.12 | 1,5<br>6 | 66,4<br>63,4 |
| 1.14 | 1,5<br>6 | 60,4<br>60,4 |

Tabelle 13

| Sommerweizen, Sorte "Ralle" Nachauflauf-Blattbehandlung | | |
|---|---|---|
| Nr. d. chem. Beispiele | Konzentration mg WS/Gefäß | relative Wuchshöhen |
| unbehandelt<br>"A"<br>2.1 | -<br>6<br>6 | 100<br>80,6<br>68,8 |

Tabelle 14

| Sommergerste, Sorte "Aramir" Nachauflauf-Blattbehandlung | | |
|---|---|---|
| Nr. d. chem. Beispiele | Konzentration mg WS/Gefäß | relative Wuchshöhen |
| unbehandelt | - | 100 |
| "A" | 1,5<br>6 | 93,2<br>87,7 |
| 2.1 | 1,5<br>6 | 63,0<br>49,3 |

Tabelle 15

| Sonnenblumen, Sorte "Spanners Allzweck" Nachauflauf-Blattbehandlung | | |
|---|---|---|
| Nr. d. chem. Beispiele | Konzentration mg WS/Gefäß | relative Wuchshöhen |
| unbehandelt | - | 100 |
| "A" | 6 | 82,2 |
| 2.2 | 6 | 72,0 |
| 2.4 | 6 | 73,2 |
| 2.5 | 6 | 73,2 |
| 2.10 | 6 | 76,2 |

Tabelle 16

| Reis, Sorte "Bahia" Nachauflauf-Blattbehandlung | | |
|---|---|---|
| Nr. d. chem. Beispiele | Konzentration mg WS/Gefäß | relative Wuchshöhen |
| unbehandelt | - | 100 |
| "A" | 1,5<br>6 | 99,7<br>94,1 |
| 2.1 | 1,5<br>6 | 91,4<br>63,7 |

Tabelle 17

| Mais, Sorte "Inrakorn" Nachauflauf-Blattbehandlung | | |
|---|---|---|
| Nr. d. chem. Beispiele | Konzentration mg WS/Gefäß | relative Wuchshöhen |
| unbehandelt | - | 100 |
| "A" | 3<br>6 | 93,1<br>93,1 |
| 2.1 | 3<br>6 | 66,8<br>62,7 |

## Patentansprüche

**1.** Azetidinderivate der allgemeinen Formel I

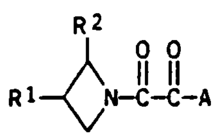

(I),

worin

$R^1$ und $R^2$ für Wasserstoff oder $C_1$-$C_3$-Alkyl und
A für die Gruppe -$XR^3$ oder -$N(R^5)$-$OR^4$ stehen, worin
X Sauerstoff oder Schwefel,
$R^3$ $C_1$-$C_{20}$-Alkyl, $C_1$-$C_{20}$-Halogenalkyl, $C_3$-$6_{18}$-Alkenyl, $C_3$- oder $C_4$-Alkinyl, ggf. überbrücktes $C_3$-$C_{12}$-Cycloalkyl oder $C_4$-$C_{12}$-Alkylcycloalkyl, wobei die Cycloalkylreste

bzw. Alkylcycloalkylreste durch $C_1$-$C_5$-Alkyl oder Cyclohexyl substituiert sein können,

$R^4$ $C_1$-$C_{20}$-Alkyl, $C_1$-$C_{12}$-Halogenalkyl, $C_1$-$C_6$-Cyanalkyl, $C_1$-$C_{12}$-Alkoxyalkyl, $C_3$-$C_{18}$-Alkenyl, $C_3$-$C_{18}$-Halogenalkenyl, $C_3$- oder $C_4$-Alkinyl, $C_3$-$C_{12}$-Cycloalkyl oder $C_4$-$C_{12}$-Cycloalkylalkyl, $C_3$-$C_{16}$-Alkoxycarbonylalkyl oder jeweils gegebenenfalls substituiertes $C_7$-$C_{20}$-Aralkyl und

$R^5$ Wasserstoff oder $C_1$-$C_3$-Alkyl

bedeuten, ausgenommen N-Methoxyoxalyl-azetidin.

**2.** Verfahren zur Herstellung von Azetidinderivaten der allgemeinen Formel I gemäß Anspruch 1, in der A für -$XR^3$ steht, dadurch gekennzeichnet, daß man

a) Azetidine der Formel II

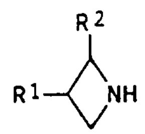

(II)

mit einem Oxalesterchlorid der Formel III

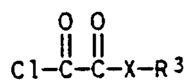

(III),

wobei $R^1$, $R^2$, X und $R^3$ die oben genannte Bedeutung haben, oder

b) ein Oxalamidchlorid der Formel IV

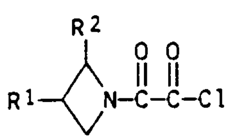

(IV)

mit einem Alkohol oder Thiol der Formel V

HX-$R^3$ (V),

wobei $R^1$, $R^2$, X und $R^3$ die oben angegebenen Bedeutungen haben, gegebenenfalls in Gegenwart eines Lösungs- oder Verdünnungsmittels und/oder einer anorganischen oder organischen Base und/oder eines Reaktionsbeschleunigers umsetzt.

**3.** Verfahren zur Herstellung von Azetidinderivaten der allgemeinen Formel I gemäß Anspruch 1, in der A für -N($R^5$)-O$R^4$ steht, dadurch gekennzeichnet, daß man

a) Oxalamidchloride der Formel IV

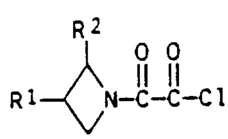

(IV)

mit einem Hydroxylamin-Derivat der Formel VI

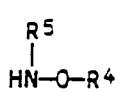

(VI)

wobei $R^1$, $R^2$, $R^4$ und $R^5$ die oben angegebene Bedeutung haben, oder
b) ein Oxalamidester der Formel VII

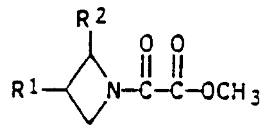

(VII)

mit einem Hydroxylamin-Derivat der Formel VI

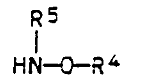

(VI)

wobei $R^1$, $R^2$, $R^4$ und $R^5$ die oben angegebenen Bedeutungen haben, gegebenenfalls in Gegenwart eines Lösungs- oder Verdünnungsmittels und/oder einer anorganischen oder organischen Base und/oder eines Reaktionsbeschleunigers umsetzt.

4. Mittel zur Regulierung des Pflanzenwachstums, enthaltend ein Azetidinderivat der Formel I gemäß Anspruch 1 und übliche inerte Zusatzstoffe.

5. Mittel zur Regulierung des Pflanzenwachstums, enthaltend 0,1 bis 95 Gew.-% eines Azetidinderivats der Formel I gemäß Anspruch 1 und übliche inerte Zusatzstoffe.

6. Mittel zur Regulierung des Pflanzenwachstums, enthaltend N-Methoxyoxalylacetidin und übliche inerte Zusatzstoffe.

7. Mittel zur Regulierung des Pflanzenwachstums, enthaltend 0,1 bis 95 Gew.-% N-Methoxyoxalylacetidin und übliche inerte Zusatzstoffe.

8. Verfahren zur Regulierung des Pflanzenwachstums, dadurch gekennzeichnet, daß man den Boden, die Pflanzen oder ihren Samen mit einem Azetidinderivat der Formel I gemäß Anspruch 1 oder N-Methoxyoxalylazetidin behandelt.

9. Verfahren zur Regulierung des Pflanzenwachstums, dadurch gekennzeichnet, daß man mindestens ein Azetidinderivat der Formel I gemäß Anspruch 1 oder N-Methoxyoxalylazetidin oder ein Mittel nach den Ansprüchen 4, 5, 6 oder 7 auf Pflanzen oder deren Lebensraum einwirken läßt.

## Claims

1. An azetidine derivative of the formula I

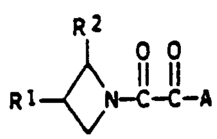

(I),

where $R^1$ and $R^2$ are each hydrogen or $C_1$-$C_3$-alkyl, A is $-XR^3$ or $-N(R^5)$ $-OR^4$, X is oxygen or sulfur, $R^3$ is $C_1$-$C_{20}$-alkyl, $C_1$-$C_{20}$-haloalkyl, $C_3$-$C_{18}$-alkenyl, $C_3$- or $C_4$-alkynyl, $C_3$-$C_{12}$-cycloalkyl or $C_4$-$C_{12}$-alkylcycloalkyl which may be bridged, it being possible for the cycloalkyl radicals or alkylcycloalkyl radicals to be substituted by $C_1$-$C_5$-alkyl or cyclohexyl, $R^4$ is $C_1$-$C_{20}$-alkyl, $C_1$-$C_{12}$-haloalkyl, $C_1$-$C_6$-cyanoalkyl, $C_1$-$C_{12}$-alkoxyalkyl, $C_3$-$C_{18}$-alkenyl, $C_3$-$C_{18}$-haloalkenyl, $C_3$- or $C_4$-alkynyl, $C_3$-$C_{12}$-cycloalkyl or $C_4$-$C_{12}$-cycloalkylalkyl, $C_3$-$C_{16}$-alkoxycarbonylalkyl or unsubstituted or substituted $C_7$-$C_{20}$-aralkyl and $R^5$ is hydrogen or $C_1$-$C_3$-alkyl, with the exception of N-methoxyoxalylazetidine.

**2.** A process for the preparation of an azetidine derivative of the formula I as claimed in claim 1, where A is $-XR^3$, which comprises reacting
a) an azetidine of the formula II

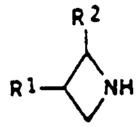

(II)

with an oxalic ester chloride of the formula III

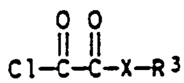

(III),

where $R^1$, $R^2$, X and $R^3$ have the abovementioned meanings,
or
b) an oxalamide chloride of the formula IV

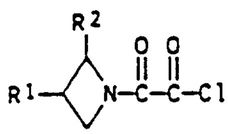

(IV)

with an alcohol or thiol of the formula V

$HX\text{-}R^3$ (V)

where $R^1$, $R^2$, X and $R^3$ have the abovementioned meanings, in the presence or absence of a solvent or diluent and/or of an inorganic or organic base and/or of a reaction accelerator.

**3.** A process for the preparation of an azetidine derivative of the formula I as claimed in claim 1, where A is $-N(R^5)\text{-}OR^4$, which comprises reacting
a) an oxalamide chloride of the formula IV

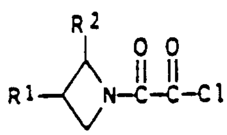

(IV)

with a hydroxylamine derivative of the formula VI

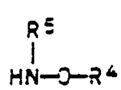

(VI)

where $R^1$, $R^2$, $R^4$ and $R^5$ have the abovementioned meanings, or

b) an oxalamide ester of the formula VII

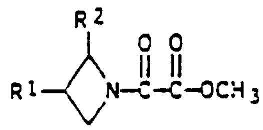

(VII)

with a hydroxylamine derivative of the formula VI

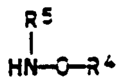

(VI)

where $R^1$, $R^2$, $R^4$ and $R^5$ have the abovementioned meanings, in the presence or absence of a solvent or diluent and/or of an inorganic or organic base and/or of a reaction accelerator.

**4.** An agent for regulating plant growth, containing an azetidine derivative of the formula I as claimed in claim 1 and conventional inert additives.

**5.** An agent for regulating plant growth, containing from 0.1 to 95 % by weight of an azetidine derivative of the formula I as claimed in claim 1 and conventional inert additives.

**6.** An agent for regulating plant growth, containing N-methoxyoxalylazetidine and conventional inert additives.

**7.** An agent for regulating plant growth, containing from 0.1 to 95 % by weight of N-methoxyoxalylazetidine and conventional inert additives.

**8.** A method for regulating plant growth, wherein the soil, the plants or their seeds are treated with an azetidine derivative of the formula I as claimed in claim 1 or with N-methoxyoxalylazetidine.

**9.** A method for regulating plant growth, wherein at least one azetidine derivative of the formula I as claimed in claim 1 or N-methoxyoxalylazetidine or an agent as claimed in any of claims 4, 5, 6 or 7 is allowed to act on plants or their habitat.

## Revendications

**1.** Dérivés d'azétidine de formule générale I

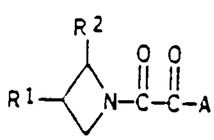

(I),

dans laquelle
$R^1$ et $R^2$ sont mis pour hydrogène ou alkyle en C1-C3 et
A pour le groupe $-XR^3$ ou $-N(R^5)-OR^4$ où
X représente oxygène ou soufre,
$R^3$ représente alkyle en C1-C20, halogènalkyle en C1-C20, alcényle en C3-C18, alcynyle en C3 ou C4, cycloalkyle en C3-C12 ou alkylcycloalkyle en C4-C12 éventuellement pontés, les restes cycloalkyle ou alkylcycloalkyle pouvant être substitués par alkyle en C1-C5 ou cyclohexyle,
$R^4$ représente alkyle en C1-C20, halogène-alkyle en C1-C12, cyanalkyle en C1-C6, alcoxyalkyle en C1-C12, alcényle en C3-C18, halogènealcényle en C3-C18, alcynyle en C3 ou C4, cycloalkyle en C3-C12 ou cyclalkylalkyle en C4-C12, alcoxycarbonylalkyle en C3-C16 ou aralkyle en C7-C20, dans chaque cas

éventuellement substitué, et
$R^5$ représente hydrogène ou alkyle en C1-C3,
N-méthoxyoxalyl-azétidine étant exceptée.

**2.** Procédé de préparation de dérivés d'azétidine de formule générale I, selon la revendication 1, dans laquelle A est mis pour $-XR^3$, caractérisé par le fait que l'on fait réagir

a) des azétidines de formule II

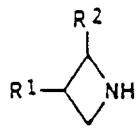

(II)

avec un chlorure d'ester oxalique de formule III

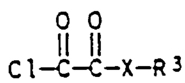

(III),

$R^1$, $R^2$, X et $R^3$ ayant les significations données plus haut, ou

b) un chlorure d'oxalamide de formule IV

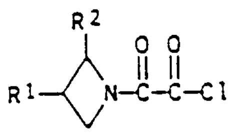

(IV)

avec un alcool ou thiol de formule V

$HX-R^3$ (V),

où $R^1$, $R^2$, X et $R^3$ ont les significations données plus haut, éventuellement en présence d'un solvant ou diluant et/ou d'une base inorganique et/ou d'un accélérateur de réaction.

**3.** Procédé de préparation de dérivés d'azétidine de formule générale I, selon la revendication 1, dans laquelle A est mis pour $-N(R^5)-OR^4$, caractérisé par le fait que l'on fait réagir

a) des chlorures d'oxalamide de formule IV

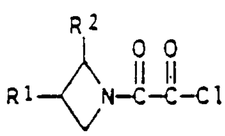

(IV)

avec un dérivé d'hydroxylamine de formule VI

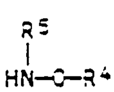

(VI)

$R^1$, $R^2$, $R^4$ et $R^5$ ayant les significations données plus haut, ou

b) un ester d'oxalamide de formule VII

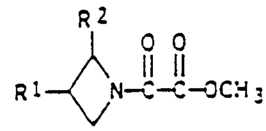

(VII)

avec un dérivé d'hydroxylamine de formule VI

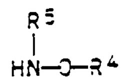

(VI)

où $R^1$, $R^2$, $R^4$ et $R^5$ ont les significations données plus haut, éventuellement en présence d'un solvant ou diluant et/ou d'une base inorganique et/ou d'un accélérateur de réaction.

**4.** Agent pour régulariser la croissance des plantes, contenant un dérivé d'azétidine de formule I, selon la revendication 1, et des additifs inertes usuels.

**5.** Agent pour régulariser la croissance des plantes, contenant 0,1 à 95 % en poids d'un dérivé d'azétidine de formule I, selon la revendication 1, et des additifs inertes usuels.

**6.** Agent pour régulariser la croissance des plantes, contenant de la N-méthoxyoxalylacétidine et des additifs inertes usuels.

**7.** Agent pour régulariser la croissance des plantes, contenant 0,1 à 95 % en poids de N-méthoxyoxalylacétidine et des additifs inertes usuels.

**8.** Procédé pour régulariser la croissance des plantes, caractérisé par le fait que l'on traite le sol, les plantes ou leurs semences avec un dérivé d'azétidine de formule I, selon la revendication 1, ou de la N-méthoxyoxalylazétidine.

**9.** Procédé pour régulariser la croissance des plantes, caractérisé par le fait que l'on fait agir, sur des plantes ou leur biotope, au moins un dérivé d'azétidine de formule I, selon la revendication 1, ou de la N-méthoxyoxalylazétidine ou un agent selon l'une des revendications 4, 5, 6 ou 7.